# EUROPEAN PATENT SPECIFICATION

(11) **EP 1 318 792 B1**
(45) Date of publication and mention of the grant of the patent: **05.01.2005**
(21) Application number: 01971528.3
(22) Date of filing: 21.09.2001
(51) Int. Cl.: A61K 9/50, A61K 31/7048

(54) **SUSTAINED RELEASE COMPOSITION CONTAINING CLARITHROMYCIN**
CLARITHROMYCIN ENTHALTENDE ZUBEREITUNG MIT VERLÄNGERTER FREISETZUNG
COMPOSITION A LIBERATION PROLONGEE CONTENANT DE LA CLARITHROMYCINE

(30) Priority: 22.09.2000 WO PCT/BE00/00112
(43) Date of publication of application: 18.06.2003
(73) Proprietor: Galephar M/F, 6900 Marche-en-Famenne (BE)
(72) Inventor: VANDERBIST, Francis, B-1170 Bruxelles (BE); SERENO, Antonio, B-1820 Melsbroek (BE); BAUDIER, Philippe, B-1180 Uccle (BE)
(74) Representative: Powis de Tenbossche, Roland
(86) International application number: PCT/BE2001/000164
(87) International publication number: WO 2002/024174

(56) References cited:
- EP-A- 0 293 885
- WO-A-01/35930
- WO-A-01/62195
- WO-A-95/22319
- WO-A-98/47493

## Description

### ABSTRACT

Disclosed is a method of treating infection including a sustained release oral form of CLARITHROMYCIN constituted by coated pellets and allowing a once a day administration of the drug.

### BACKGROUND OF THE INVENTION

Clarithromycin is a semisynthetic macrolide antibiotic derived from erythromycin. Clarithromycin is primarily bacteriostatic, it exerts its antimicrobial effect by the inhibition of protein synthesis on bacterial ribosomes. Clarithromycin is active against the major pathogens responsible for respiratory tract infections in immunocompetent patients, namely Chlamydia pneumoniae, Mycoplasma pneumoniae, Staphylococcus aureus, Streptococcus pyogenes, Moraxella cathanhallis, Streptococcus pneumoniae, Haemophilis influenzae. Clarithromycin is also active against and Helicobacter pylori.

Clarithromycin is rapidly absorbed and its availability after an oral dose of 250 mg is approimatively 55 %. This is probably due to the first-pass metabolism, which produces, in particular, the 14-hydroxy active metabolite. It has been shown that the maximal serum concentration following oral administration are dose dependent and the time to achieve peak blood concentrations is about 2 hours.

There is an effect of food on the bioavailability of clarithromycin and 14- hydroxy-clarithromycin. The food intake immediately before administration increases the bioavailability by a mean of 25%. Such an increase can be considered of little clinical significance with the dosage regimen of 250 and 500 mg twice daily.

Macrolides antibiotics are lipid soluble and extensively distributed both in body fluids and tissues. Clarithromycin also achieves tissue concentrations markedly higher than circulating levels, due to its wide distribution. This aspect is relevant for clinical activity.

Clinical trials in adults have shown similar efficacy for clarithromycin and other antibacterial drugs in the treatment of community-acquired pneumonia, acute bronchitis, acute exacerbations of chronic bronchitis.
Comparators agents included the β-lactam agents anpicillin, amoxicillin with or without clavulanic acid, penicillin V, some Cephalosporins (cefaclor, cefuroxime, ...) and the other macrolides erythromycin, roxithromycin, azithromycin.
The most usual way of oral administration of clarithromycin to the adults is an immediate release tablet to be taken twice daily.
A modified release formulation of clarithromycin has been developed to allow administration of the drug once daily. This formulation delivers the same peak and through concentrations of the parent drug and metabolite and reaches equivalent AUC values to those seen with the twice daily immediate-release formulation in the 24 hours after administration. The elimination half life of clarithromycin and its 14-hydroxymetabolite are unaltered by the formulation although peak plasma concentrations are delayed with the once-daily dosage form.

Some developments trials have been made to obtain a sustained-release of clarithromycin after oral administration, for instance :
- Patent 6,010,718 describes a pharmaceutical composition for extended release of an erythromycin derivative in the gastrointestinal environment. The composition comprises an erythromycin derivative and a pharmaceutically acceptable polymer so that, when ingested orally, the composition induces significantly lower Cₘₐₓ in the plasma than an immediate release composition of the erithromycin derivative while maintaining bioavailability and minimum concentration substancially equivalent to that of the immediate release composition of the erithromycin derivative upon multiple dosing. The compositions of the invention have an improved taste profile and reduced gastrointestinal side effects as compared to those for the immediate release composition.
- Patent 5,705,190 describes a controlled release, oral, solid, pharmaceutical composition for a reduced daily dosage regimen, where the therapeutic ingredient is a poorly soluble basic drug. The formulation comprises the use of a water-soluble alginate salt, a complex salt of alginic acid and an organic carboxylic acid in admixture with the therapeutic drug. A particular embodiment comprising a once a day dosage form for clarithromycin is also described.
- Patent 5,051,262 describes an invention which specifically relates to processes for preparing delayed action galenic forms. The process is characterized in that the application solutions of excipients, coatings and active constituents are adjusted to a desired pH. The independence of the rate of dissolution of a controlled release or sustained action oral pharmaceutical form is increased by admixing a pH adjusting agent with every application solution of medicament, excipient or coating, throughout the course of formulation of the pharmaceutical form.
- Patent WO 98/47493 describes a pharmaceutical formulation which is provided in powder form by spray-drying to form a polymeric coated core element which coating both masks the taste of the active ingredient present in the core and provide sustained release properties.

### BRIEF DESCRIPTION OF THE INVENTION

The present invention relates to a sustained release form of clarithromycin consisting in coated pellets, whereby an once daily administration of the drug is possible.

The pharmaceutical oral sustained release composition of clarithromycin of the invention contains clarithromycin coated pellets comprising each a core containing at least 50% by weight of clarithromycin and a sustained release coating surrounding the core, in which the sustained release coating comprises at least a water insoluble polymer which is substantially pH independent at least for a pH range comprised between 2 and 7, advantageously for a pH range from about 2 to 7.5, preferably from 1.5 to 8, most preferably from about 1 to 8, whereby the amount of the sustained release coating corresponds from 6 to 20% of the weight of clarithromycin containing core and in which the sustained release coating has a thickness comprised between 30 and 200 µm. A water insoluble polymer which is substantially independent at least at pH comprised between 2 and 7.5 is a polymer allowing substantially the same rate of passage of clarithromycin in said pH range.

The core of the clarithromycin containing pellets is advantageously manufactured using the process of extrusion-spheronization.
The water insoluble polymer which is substantial pH independent is advantageously selected from the group consisting of acrylic polymers, methacrylic polymers, acrylic copolymers, methacrylic copolymers, acrylic-methacrylic copolymers, cellulosic derivatives, and mixtures thereof.
in a preferred embodiment, the water insoluble polymer is an ethyl acrylate and methyl methacrylate neutral copolymer. According to another preferred embodiment, the water insoluble polymer is a cellulosic derivative.

The sustained release coating contains for example from 1 to 50% by weight, but preferably from 5 to 20% by weight of water insoluble polymer which is substantially pH independent. For example, the sustained release coating contains from 5 to 20% by weight of water insoluble acrylic polymer or copolymer or cellulosic derivative which is substantially pH independent.

The clarithromycin containing core is coated with an amount of the sustained release coating corresponding from 6% to 20% of the weight of the clarithromycin containing core. The clarithromycin containing core contains advantageously from 5 to 50% by weight of microcrystalline cellulose and/or from 0.5% to 5% by weight of polyvinylpyrolidose and/or from 2 and 20% of one or more organic acids. According to a preferred embodiment, the clarithromycin containing core contains microcrystalline cellulose, one or more citric acid and possibly, but preferably, polyvinylpyrolidose
The sustained release coating has advantageously a thickness between about 30 and 200 µm, advantageously between 30 and 150µm, for example about 50µm, about 75 µm, about 100µm, about 150µm. According to an advantageous embodiment, the thickness of the sustained release coating is substantially constant. For example the thickness varies essentially in a range of -25% to +25% with respect to the average thickness, advantageously in a range of -15% to +15% with respect to the average thickness, preferably in a range of -10% to +10% with respect to the average thickness.
The pellets have preferably a size between 0.5 and 2.0 mm.
The invention relates also to a pharmaceutically acceptable capsule containing pellets as described here above in the composition of the invention.
The pharmaceutical capsule is for example a soft gelatine capsule, but preferably a hard gelatine capsule.
The pharmaceutical capsule of the invention contains advantageously from 100 to 500 mg of clarithromycin in the form of pellets of the invention.

The pharmaceutical capsule contains preferably a sufficient amount of clarithromicyn coated pellets of the invention for having an effective antiinfective effect when administering once daily the patient.

With compositions of the invention and capsules of the invention, it is possible to ensure a low maximal concentration in order to decrease the frequence of side effects associated with the intake of clarithromycin.
With compositions of the invention and capsules of the invention, it is possible to ensure a decrease of the intra- and intersubjects variability of the plasma concentration after an oral intake of clarithromycin pellets.

### DESCRIPTION OF THE DRAWINGS

FIGURE 1 shows the Influence of the amount of film coating on the in vitro dissolution rate of clarithromycin (n=6 vessels/ test) ;
FIGURE 2 shows a mean pharmacokinetic profile after a multiple dose of 500 mg clarithromycin pellets administered once daily (n=8 subjects).

### DESCRIPTION OF PREFERRED EMBODIMENTS

Pellets are spheres of varying diameter depending on the application and the wish of the producer. Most often in the pharmaceutical industry the size of the pellets is 0.5-2.0 mm.
Pellets as a drug delivery system offer not only therapeutical advantages such as less irritation of the gastro-intestinal tract, a lowered risk of side effects due to dose dumping and bioavailability less dependent on the food intake but also technological advantages, for example, better flow properties, less friable dosage form, narrow particle size distribution, ease of coating and uniform packing.
The reproducibility of the drug blood levels is an additional advantage to the use of a pellet formulation. Pellets are commonly filled into hard gelatine capsules, but can also be compressed to tablets.

Although pellets can be produced in different ways (spraying a solution or suspension onto an inert core, building the pellet layer after layer, spray-drying a solution or a suspension of the drug forming pellets due to the evaporation of fluid phase,...), the most popular method of manufacturing is by the extrusion-spheronisation technique.
This process involves at least five steps : blending-preparation of the net mass (granulation), shaping the net mass into cylinders (extrusion), breaking up the extrudate and rounding of the particles into spheres (spheronisation) and finally drying of the pellets.
It has been found that by using a sustained release coating containing a water insoluble polymer, which is substantially pH independent, that the dissolution rate of clarithromycin could be controlled, at pH below 5 (where the water solubility of clarithromycin is good and quite constant), as well as at higher pH , such as at pH comprised between 5 and 7 (the solubility of clarithromycin decreases dramatically at pH greater than 5 and becomes quasi nil at pH=8). The sustained release coating of the invention controls therefore the release of clarithromycin in the small intestine where the pH is between 5.5 and 7.0. Furthermore, at acidic pH (1.4), the stability of clarithromycin is not optimal. Indeed, the half-life of decomposition of clarithromycin is of 17 minutes at pH = 1.4.

The core pellets of the invention contain preferably more than 50% (w/w) of clarithromycin. The excipients used to allow the manufacture of the pellets include but one restricted to : microcrystalline cellulose, polyvinylpyrrolidose, hypromellose, surcrose stearate, citric acid, stearic acid, lactose and other mono- or disaccharrides. In particular, it should be ensured that the excpients used always guarantee an optimal dissolution of clarithromycin.

The granulation is done using a hydro ethanolic solution in which the ratio between water and ethanol varies between 1/50 (w/w) and 1/2 (w/w). This granulating liquid allows to obtain the most suitable mass for the subsequent extrusion-spheronisation process. Indeed, the use of water alone as granulating liquid provokes the formation of a mass too sticky to allow a good extrusion-spheronisation process.

To allow a good extrusion process, the steps of blending and granulation must be performed in a way that allows to prevent the evaporation of the granulation liquid in order to avoid that the granulate mass becomes too dry to be extruded. The granulation-extrusion steps must be performed in a special apparatus which allows the granulation and extrusion as a continuous step. Indeed, the granulator is equipped with a special output, allowing the extrusion once the mass possess the adequate extrusion properties. The granulating tank is also equipped with an airthight cover to prevent evaporation of the granulating liquid.

The coating process of the pellets may be performed, for instance, using the fluid bed coater technology.
To guarantee a continuous release and dissolution of clarithromycin, polymer coating must have properties such as it allows a release of the drug which is independent to the pH. The most suitable polymers for the purpose and which are pharmaceutically acceptable are the family of neutral acrylic derivatives and the water insoluble cellullosic derivatives such as ethylcellullose.

### EXAMPLES

Some Examples of formulations for the core pellets and the coatings of the pellets are given hereinbelow.

### Formulations

| **Core pellets** | | | | | |
|---|---|---|---|---|---|
| Ingredients | F1 | F2 | F3 | F4 | F5 |
| Clarithromycin | 60 | 72 | 60 | 60 | 60 |
| Microcrystalline cellullose | 19 | 26 | 34 | 19 | 19 |
| Povidone | 2 | 2 | --- | --- | 2 |
| Citric acid Trihydrate | 14 | --- | --- | --- | 19 |
| Stearic acid | 5 | --- | --- | --- | --- |
| Sucrose stearate | --- | --- | 4 | --- | --- |
| hypromellose | --- | --- | 2 | 2 | --- |
| Lactose | --- | --- | --- | 19 | --- |

The pellets or microgranules had a size comprised between 0.5 mm and about 2 mm.

| ***Coatings*** | | | |
|---|---|---|---|
| | C1 | C2 | C3 |
| Polyacrylate dispersion 30 % (Dry residue) | 65.6 | --- | --- |
| Ammonio methacrylate copolymer | --- | 64.83 | --- |
| Ethylcellulose | --- | --- | 64.8 |
| Polysorbate 80 | 0.15 | --- | --- |
| Siméthicone emulsion | 1.46 | 1.50 | --- |
| Hypromellose | 10.93 | 7.54 | --- |
| Talc | 14.58 | 15.07 | 22.61 |
| Titanium dioxyde | 7.28 | 7.54 | 7.54 |
| Triacetin | --- | --- | 5.03 |
| Triethyl citrate | --- | 3.52 | --- |

The coatings C1,C2,C3 can be applied on anyone of the core pellets F1 to F5.
The thickness of the coating on the pellets was about 30-200 µm.

The dissolution test is usually the most appropriate analytical tool to assess the quality of the oral formulations and especially of sustained release oral formulations.

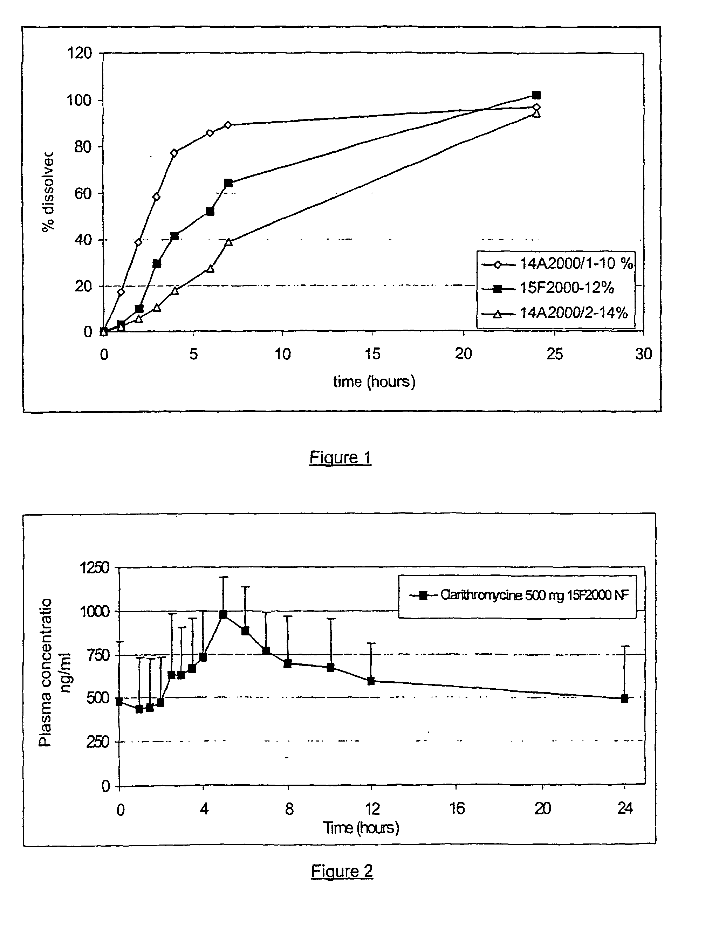

The conditions used for assessing the dissolution rate of clarithromycin are the following:
□ Paddle Apparatus (EP, 3^{rd} edition, 2.9.3, figure 1)
□ pH 5.0 (phosphate buffer)
□ Rotation speed of the paddles : 100 rpm
□ Detection : HPLC (UV detection) (wavelength 286 nm)
□ Volume of dissolution liquid : 900 ml
The figure 1 hereinbelow shows the influence of the amount of film coating on the dissolution rate of clarithromycin

FIGURE 1 shows the Influence of the amount of film coating on the in vitro dissolution rate of clarithromycin (n=6 vessels/ test).

Logically, the dissolution rate of darithomycin decreases when the amount of film coating increases. As it can be seen from said figure, the % of dissolved clarithromycin was about 80% after about 5 minutes when using an amount of coating corresponding to 10% of the weight of the core, while said % of dissolved darithromicin after 5 minutes was respectively about 40% and about 20% when using respectiveley an amount of coating corresponding to 12% and 14% of the weight of the core.

An in vivo, multiple dose pharmacokinetic study has been performed on 8 healthy volunteers to assess the bioavailability of the compositions relative to the present invention.
The mean pharmacokinetic profile obtained is given in figure 2 [ Mean pharmacokinetic profile after a multiple dose of 500 mg clarithromycin pellets administered once daily (n=8 subjects)]. The tested patients received a hard gelatine capsule containing coated pellets corresponding to 500mg clarithromicyn.

As it can be seen from figure 2, by using the composition of the invention it is possible to provide a sustained release once a day formulation of clarithromycin. The said formulation being efficient to treat or prevent infections and presenting a more favourable profile of side effects than the existing inmmediate release tablet and than the existing sustained release tablets.

This safer profile is due to a lower Cₘₐₓ of clarithromycin than the references after a multiple dose administration of clarithromycin. For comparison, the Cₘₐₓ obtained after a multiple dose administration of the the reference BICLAR 250 mg is of 1.0 µg/ml. For BICLAR 500 mg, the Cₘₐₓ obtained after a multiple dose administration is of 2.7 µg/ml . The formulation relative to the invention clearly provides lower Cₘₐₓ than the immediate release formulations of clarithromycin. Moreover, it is clear from figure 2, that release of clarithromycin allows to obtain effective plasmatic concentration of clarithromycin 24 hours after the intake.

By using the composition of the invention, it is also possible to obtain lower intra and inter individual variability than the commercialized forms of clarithromycin. The variability obtained in the pharmacokinetic study described in figure 2 is low.

## Claims

1. A pharmaceutical oral sustained release composition of clarithromycin containing coated pellets comprising each a core containing at least 50% by weight of clarithromycin and a sustained release coating surrounding the core, in which the sustained release coating comprises at least a water insoluble polymer which is substantial pH independent at least for a pH range from 2 to 7, whereby the amount of the sustained release coating corresponds from 6 to 20% of the weight of clarithromycin containing core and in which the sustained release coating has a thickness comprised between 30 and 200µm.

2. The pharmaceutical composition of claim 1, in which the pellets have a size between 0.5 mm and 2 mm.

3. The pharmaceutical composition of claim 1, in which the core of the clarithromycin containing pellets is manufactured using the process of extrusion-spheronization.

4. The pharmaceutical composition of claim 1, wherein the core of the clarithromycin containing pellets contains between 5 and 50% by weight of microcrystalline cellulose.

5. The pharmaceutical composition of claim 1, wherein the water insoluble polymer which is substantially pH independent is selected from the group consisting of acrylic polymers, methacrylic polymers, acrylic copolymers, methacrylic copolymers, acrylic-methacrylic copolymers, cellulosic derivatives, and mixtures thereof.

6. The pharmaceutical composition of claim 1, wherein the water insoluble polymer is an ethyl acrylate and methyl methacrylate neutral copolymer.

7. The pharmaceutical composition of claim 1, in which the sustained release coating contains from 5 to 20% by weight of water insoluble polymer which is substantially pH independent.

8. The pharmaceutical composition of claim 1, in which the sustained release coating contains from 5 to 20% by weight of water insoluble acrylic polymer or copolymer which is substantially pH independent.

9. The pharmaceutical composition of claim 1, wherein the clarithromycin containing core is coated with an amount of the sustained release coating corresponding from 6% to 20% of the weight of the clarithromycin containing core.

10. The pharmaceutical composition of claim 1. wherein the core of the clarithromycin containing pellets contains between 0.5% and 5% by weight of polyvinylpyrolidose

11. The pharmaceutical composition of claim 1, wherein the core of the clarithromycin containing pellets contains between 2 and 20% of one or more organic acids.

12. The pharmaceutical composition of claim 1, wherein the sustained release coating has a thickness between 30 and 200 µm

13. The pharmaceutical composition of claim 1, wherein the water insoluble polymer is a cellulosic derivative.

14. The pharmaceutical composition of claim 1, which contains an effective amount of clarithromycin for ensuring when administering the composition once daily, an effective antiinfective effect during one day.

15. A pharmaceutically acceptable capsule containing pellets of anyone of the claims 1 to 14.

16. The pharmaceutical capsule of claim 15, where the capsule is a hard gelatine capsule.

17. The pharmaceutical capsule of claim 15, which contains from 100 to 500 mg of clarithromycin in the form of pellets of anyone of the claims 1 to 14.

## Patentansprüche

1. Pharmazeutische oral zu verabreichende langzeitwirkende Zusammensetzung von Clarithromycin, bestehend aus beschichteten Kügelchen, von denen jedes aus einem Kern besteht, der mindestens 50 Gewichts-% Clarithromycin enthält, und aus einer langzeitwirkenden, den Kern umhüllenden Beschichtung, wobei die langzeitwirkende Beschichtung aus mindestens einem in Wasser unlöslichen Polymer besteht, das im wesentlichen pH-unabhängig ist, zumindest in einem pH-Bereich von 2 bis 7, wobei die Menge der langzeitwirkenden Beschichtung 6 bis 20 % des Gewichts des Clarithromycin enthaltenden Kerns entspricht, und die langzeitwirkende Beschichtung eine Dicke zwischen 30 und 200 mµ aufweist.

2. Pharmazeutische Zusammensetzung nach Anspruch 1, bei der die Kügelchen eine Größe zwischen 0,5 mm und 2 mm besitzen.

3. Pharmazeutische Zusammensetzung nach Anspruch 1, bei der der Kern der Clarithromycin enthaltenden Kügelchen nach dem Extrusions-Sphäronisations-Verfahren hergestellt ist.

4. Pharmazeutische Zusammensetzung nach Anspruch 1, worin der Kern der Clarithromycin enthaltenden Kügelchen zwischen 5 und 50 Gewichts-% mikrokristalline Zellulose enthält.

5. Pharmazeutische Zusammensetzung nach Anspruch 1, worin das in Wasser unlösliche Polymer, das im wesentlichen pH-unabhängig ist, aus der Gruppe ausgewählt ist, die aus Acryl-Polymeren, Methacryl-Polymeren, Acryl-Copolymeren, Methacryl-Copolymeren, Acryl-Methacryl-Copolymeren, Zellulose-Derivaten und Mischungen daraus besteht.

6. Pharmazeutische Zusammensetzung nach Anspruch 1, worin das in Wasser unlösliche Polymer ein neutrales Ethylacrylat- und Methylmethacrylat-Copolymer ist.

7. Pharmazeutische Zusammensetzung nach Anspruch 1, bei der die langzeitwirkende Beschichtung 5 bis 20 Gewichts-% eines in Wasser unlöslichen Polymers enthält, das im wesentlichen pH-unabhängig ist.

8. Pharmazeutische Zusammensetzung nach Anspruch 1, bei der die langzeitwirkende Beschichtung 5 bis 20 Gewichts-% eines in Wasser unlöslichen Acryl-Polymers oder -Copolymers enthält, das im wesentlichen pH-unabhängig ist.

9. Pharmazeutische Zusammensetzung nach Anspruch 1, worin der Clarithromycin enthaltende Kern mit einer Menge an langzeitwirkender Beschichtung versehen ist, die 6 bis 20 % des Gewichts des Clarithromycin enthaltenden Kerns entspricht.

10. Pharmazeutische Zusammensetzung nach Anspruch 1, worin der Kern der Clorithromycin enthaltenden Kügelchen zwischen 0,5 und 5 Gewichts-% Polyvinylpyrrolidon enthält.

11. Pharmazeutische Zusammensetzung nach Anspruch 1, worin der Kern der Clarithromycin enthaltenden Kügelchen zwischen 2 und 20 % einer oder mehrerer organischer Säuren enthält.

12. Pharmazeutische Zusammensetzung nach Anspruch 1, worin die langzeitwirkende Beschichtung eine Dicke zwischen 30 und 200 µm hat.

13. Pharmazeutische Zusammensetzung nach Anspruch 1, worin das in Wasser unlösliche Polymer eine Zellulose-Derivat ist.

14. Pharmazeutische Zusammensetzung nach Anspruch 1, die eine wirksame Menge Clarithromycin enthält, die, wenn die Verabreichung der Zusammensetzung 1-mal täglich erfolgt, eine wirksame infektionsverhütende Wirkung für die Dauer eines Tages sicherstellt.

15. Pharmazeutisch akzeptable Kapsel, die Kügelchen nach einem der Ansprüche 1 bis 14 enthält.

16. Pharmazeutische Kapsel nach Anspruch 15, wobei die Kapsel eine Hartgelatine-Kapsel ist.

17. Pharmazeutische Kapsel nach Anspruch 15, die 100 bis 500 mg Clarithromycin in Form von Kügelchen nach einem der Ansprüche 1 bis 14 enthält.

## Revendications

1. Composition pharmaceutique orale à libération prolongée de clarithromycine contenant des pastilles enrobées comprenant chacune un noyau contenant au moins 50% en poids de clarithromycine et un enrobage à libération prolongée entourant le noyau, dans laquelle l'enrobage à libération prolongée comprend au moins un polymère insoluble dans l'eau qui est substantiellement indépendant du pH au moins pour un intervalle de pH de 2 à 7, par quoi la quantité d'enrobage à libération prolongée correspond à 6 à 20% du poids du noyau contenant la clarithromycine et dans laquelle l'enrobage à libération prolongée a une épaisseur comprise entre 30 et 200 µm.

2. Composition pharmaceutique selon la revendication 1, dans laquelle les pastilles ont une taille entre 0,5 mm et 2 mm.

3. Composition pharmaceutique selon la revendication 1, dans laquelle le noyau des pastilles contenant la clarithromycine est fabriqué en utilisant le procédé d'extrusion-sphéronisation.

4. Composition pharmaceutique selon la revendication 1, dans laquelle le noyau des pastilles contenant la clarithromycine contient entre 5 et 50% en poids de cellulose microcristalline.

5. Composition pharmaceutique selon la revendication 1, dans laquelle le polymère insoluble dans l'eau qui est substantiellement indépendant du pH est choisi parmi le groupe constitué des polymères acryliques, des polymères méthacryliques, des copolymères acryliques, des copolymères méthacryliques, des copolymères acryliques-méthacryliques, des dérivés cellulosiques et des mélanges de ceux-ci.

6. Composition pharmaceutique selon la revendication 1, dans laquelle le polymère insoluble dans l'eau est un copolymère neutre d'acrylate d'éthyle et de méthacrylate de méthyle.

7. Composition pharmaceutique selon la revendication 1, dans laquelle l'enrobage à libération prolongée contient 5 à 20% en poids de polymère insoluble dans l'eau qui est substantiellement indépendant du pH.

8. Composition pharmaceutique selon la revendication 1, dans laquelle l'enrobage à libération prolongée contient 5 à 20% en poids de polymère ou de copolymère acrylique insoluble dans l'eau qui est substantiellement indépendant du pH.

9. Composition pharmaceutique selon la revendication 1, dans laquelle le noyau contenant la clarithromycine est enrobé avec une quantité d'enrobage à libération prolongée correspondant à 6 à 20% du poids du noyau contenant la clarithromycine.

10. Composition pharmaceutique selon la revendication 1, dans laquelle le noyau des pastilles contenant la clarithromycine contient entre 0,5% et 5% en poids de polyvinylpyrrolidone.

11. Composition pharmaceutique selon la revendication 1, dans laquelle le noyau des pastilles contenant la clarithromycine contient entre 2 et 20% d'un ou plusieurs acides organiques.

12. Composition pharmaceutique selon la revendication 1, dans laquelle l'enrobage à libération prolongée a une épaisseur d'environ 30 et 200 µm.

13. Composition pharmaceutique selon la revendication 1, dans laquelle le polymère insoluble dans l'eau est un dérivé cellulosique.

14. Composition pharmaceutique selon la revendication 1, qui contient une quantité efficace de clarithromycine pour assurer, quand on administre la composition une fois par jour, un effet anti-infection efficace durant un jour.

15. Capsule pharmaceutiquement acceptable contenant des pastilles selon l'une quelconque des revendications 1 à 14.

16. Capsule pharmaceutique selon la revendication 15, où la capsule est une capsule de gélatine dure.

17. Capsule pharmaceutique selon la revendication 15, qui contient 100 à 500 mg de clarithromycine sous la forme de pastilles selon l'une quelconque des revendications 1 à 14.
